(19) Europäisches Patentamt / European Patent Office / Office européen des brevets

(11) **EP 4 376 012 A1**

(12) **EUROPEAN PATENT APPLICATION**

(43) Date of publication:
**29.05.2024 Bulletin 2024/22**

(21) Application number: **22383137.1**

(22) Date of filing: **26.11.2022**

(51) International Patent Classification (IPC):
**G16C 20/30** (2019.01)     **G16C 20/70** (2019.01)

(52) Cooperative Patent Classification (CPC):
**G16C 20/30; G06N 3/045; G06N 3/084;**
**G06N 10/60; G16C 20/70**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Multiverse Computing S.L.**
**20014 Donostia / San Sebastián (ES)**

(72) Inventors:
• **ORÚS, Román**
  **20014 Donostia - San Sebastian (ES)**
• **JAHROMI, Saeed**
  **20014 Donostia - San Sebastian (ES)**

(74) Representative: **Elion IP, S.L.**
**Paseo Castellana, 150-4 dcha**
**28046 Madrid (ES)**

(54) **FERMIONIC TENSOR MACHINE LEARNING FOR QUANTUM CHEMISTRY**

(57)     A computer-implemented method comprising: processing a predetermined machine learning routine in the form of a tensor network that defines layers of tensors in the routine, the routine being adapted for a regression problem of fermionic systems that are molecules or chemical reactions, the routine being processed such that: each tensor of the tensor network of the predetermined machine learning routine is converted into a parity preserving tensor; and a sign swap tensor is introduced in the tensor network at each crossing of legs of different tensors in the tensor network of the predetermined machine learning routine, thereby implementing anticommutation fermionic operator; inputting a first many-body problem modeling a first fermionic system in the processed predetermined machine learning routine, the first fermionic system being a molecule or a chemical reaction; and outputting from the processed predetermined machine learning routine at least one parameter for the first fermionic system after having inputted the first many-body problem, the at least one parameter being inferred by the processed predetermined machine learning routine.

FIG. 3B

EP 4 376 012 A1

## Description

### TECHNICAL FIELD

The present disclosure relates to the field of digital processing. More specifically, the disclosure relates to the provision and/or use of machine learning with tensor networks for solving problems representing fermionic systems, thereby characterizing and determining the behavior or characteristics of such fermionic systems like e.g. molecules, chemical systems and reactions.

### BACKGROUND

Machine learning and artificial intelligence algorithms have been steadily growing in popularity as tools for solving day-to-day tasks that are either complex and/or monotonous. These tools must be adapted to the problem at hand so that they can yield good results.

A field in which these tools have seen little progress is quantum chemistry. Hence, quantum-mechanical calculations related to electronic structures of and contributions to properties of e.g. molecules, materials, systems, etc. have stalled. One reason for this is that fermionic systems have some particularities that introduce limitations and complexities to known solutions, making them unfeasible for solving quantum chemistry problems.

In this sense, the machine learning approaches based on neural networks attempting to process problems related to fermionic systems rely on the traditional description of neural networks. Such neural networks cannot provide accurate outputs because they are appropriate for spin systems, not for fermionic models, and unlike for systems with bosons, the neural networks inter alia suffer the problem that they do not account for the anticommutation of fermionic degrees of freedom.

There is interest in being able to apply machine learning and artificial intelligence algorithms to quantum chemistry problems to benefit from the capacity of such algorithms to provide solutions to problems in an efficient way while taking into account many features.

### SUMMARY

The present disclosure relates to methods, devices, systems, computer programs and computer-readable mediums intended to provide a quantum-inspired machine learning algorithm capable of solving problems related to fermionic systems. That, in turn, allows to simulate the dynamics and behavior of e.g. molecules and chemical reactions to estimate what their status will be at some point in time.

A first aspect of the disclosure relates to a computer-implemented method comprising:

processing a predetermined machine learning routine in the form of a tensor network defining layers of tensors in the routine, the predetermined machine learning routine being adapted for a regression problem of fermionic systems that are molecules or chemical reactions, preferably molecule potentials or chemical reaction velocities, the predetermined machine learning routine being processed such that:

each tensor of the tensor network of the predetermined machine learning routine is converted into a parity preserving tensor; and
a sign swap tensor is introduced in the tensor network at each crossing of legs of different tensors in the tensor network of the predetermined machine learning routine, thereby implementing anticommutation fermionic operators;

inputting a first many-body problem modeling a first fermionic system in the processed predetermined machine learning routine; and
outputting from the processed predetermined machine learning routine at least one result for the first fermionic system after having inputted the first many-body problem, the at least one result being inferred by the processed predetermined machine learning routine.

The method converts the predetermined machine learning routine into a fermionic routine to adapt it to the particularities of fermionic systems. In this sense, due to the nature of fermionic systems, more concretely the conservation of the number of fermionic particles in the systems, and the antisymmetric wave functions of the system with respect to the interchange of the position of the particles, i.e. the ordering of fermions in wave functions matters, the routine shall be configured to be capable of properly processing wave functions so that the inference made by the routine is meaningful and accurate for this type of systems.

Concerning the conservation of fermionic particles, a Hilbert space of the fermionic Hamiltonians are divided into two sectors, one with even particle number parities and another with odd particle number parities. The wave functions are thus also symmetric with respect to the particle parity operator P. Such symmetry is to be present in the routine by way of $Z_2$ symmetric wave functions, which can be implemented using $Z_2$ symmetric tensors that are parity preserving tensors, thereby fulfilling the aforesaid conservation of particles.

On the other hand, when fermions at sites e.g. $j$ and $j + 1$ interchange positions, a -1 phase factor shall be applied to the wave function, $\Psi_F$, to account for the anti-commutation of the fermionic creation and annihilation operators, i.e. $\Psi_F(x_1,x_2) = -\Psi_F(x_2,x_1)$. This consideration can be applied to the machine learning routine by processing the tensor network thereof in such a way that whenever legs of two tensors cross, a fermionic swap, namely a sign swap tensor, is introduced at the crossing conceptually.

By having $Z_2$ symmetry preserving tensors in the network and introducing swaps at each line crossing, the requirements for e.g. simulating molecular orbital model Hamiltonians are met.

Output or outputs after the last layer of the processed machine learning routine provide inferred parameter or parameters of the fermionic system under analysis, thereby making possible to, inter alia, simulate e.g. molecules, chemical reactions and chain reactions. Furthermore, the simulation of the electronic structure of the fermionic system is faster and more precise than other computational quantum mechanical modelling routines such as density functional theory, DFT, that is typically used when attempting to solve or simulate the behavior of fermionic systems.

Unlike existing approaches, which rely on one-dimensional tensor networks for solving quantum chemistry problems with algorithms such as e.g. DMRG and variations thereof, thereby missing the multi-dimensional connectivity structure of correlations in molecules, the present method relies on multi-dimensional tensor networks for the solving of quantum chemistry problems and avoids the shortcomings of those other approaches.

The defined machine learning routine serves for the solving or simulating of many-body problems associated with fermionic systems, which are of the same type, i.e. molecules or chemical reactions.

The first many-body problem is inputted in the processed routine by way of features thereof for their processing and eventual output of one or more parameters reflecting data of interest of the fermionic system.

In some embodiments, the at least one parameter comprises one or more of: a potential of a molecule of the first fermionic system, a velocity of a chemical reaction of the first fermionic system, dynamics of the first fermionic system, and one or more characteristics of at least one element taking part in the first fermionic system.

In some embodiments, the method further comprises processing the at least one parameter to:

estimate a future state of the first fermionic system; and/or
derive a configuration for the first fermionic system that alters dynamics thereof in a predetermined manner; and/or
compare the at least one parameter with one or more predetermined thresholds, and output at least one predetermined command based on the comparison.

The machine learning routine may provide data/information that is useful for controlling and/or predicting the behavior of the fermionic system under analysis. In this regard, the parameter(s) outputted by the routine may be indicative of the state of the fermionic system after some time has elapsed or after certain events have occurred, for example about a molecule, or a chemical or chain reaction; by way of example, a parameter outputted could be the ground state energy of a molecule. Additionally or alternatively, the parameter(s) may be used for establishing a different configuration for the fermionic system that can yield a superior performance, or for controlling the operation of the fermionic system by way of predetermined command(s) for devices controlling the fermionic system.

In some embodiments, the at least one parameter is processed so that at least the predetermined command is outputted, the at least one predetermined command being transmitted to a control device associated with the first fermionic system.

The control device is communicatively coupled with one or more apparatuses that influence the first fermionic system, for example the temperature thereof, the humidity thereof, the flow rate of any substance therein, etc. Therefore, the knowledge gained from the machine learning routine can be used to adjust the behavior of the fermionic system.

In some embodiments, the method further comprises, after transmitting the at least one predetermined command, inputting in the processed predetermined machine learning routine a second many-body problem modeling the first fermionic system upon at least one change is introduced or is to be introduced by the control device so that the processed predetermined machine learning routine outputs at least another parameter comprising one or more of: a potential of a molecule of the first fermionic system with the change, a velocity of a chemical reaction of the first fermionic system with the change, dynamics of the first fermionic system with the change, and one or more characteristics of at least one element taking part in the first fermionic system with the change.

A feedback loop can be provided whereby the controlled fermionic system can once again be introduced in the processed predetermined machine learning routine, according to a subsequent many-body problem that describes the fermionic system resulting from the alteration thereof owing to the predetermined command(s).

The at least another parameter(s) can then be used to establish the behavior or characteristics of the altered fermionic system. That, in turn, can be used to further alter the system, or decide not to alter it in the first place according to the predetermined command(s) if the subsequent many-body problem has been simulated without actually changing the system.

In some embodiments, the method further comprises providing the predetermined machine learning routine by processing a second many-body problem modeling a second fermionic system, the second many-body problem being decomposed or decomposable into a multi-dimensional tensor network.

The second many-body problem is used for setting up the machine learning routine so that subsequent many-body problems can be processed by the routine. To this end, the tensor network associated with the second many-body problem is provided such that it represents e.g. the low energy eigenstates of local interacting many-body Hamiltonians of the first many-body problem. This means that the tensor network decomposes the many-body wave function, namely Hamiltonian, locally and represents it in the tensor network as products of local tensors as known in the art.

The overall size of the tensor network, like the dimension of the tensor network, depends on, and is according to, the entanglement structure of the fermionic system represented by the many-body problem.

With the tensor network provided, which can be of any dimension, typically one- or two-dimensional, the machine learning routine is defined such that a plurality of layers is provided, each layer being a tensor network. The tensor networks of the different layers may be zero-dimensional, one-dimensional or multi-dimensional, with not all layers having to share the same dimension of its tensor network. The tensor networks may be connected to form the routine according to at least one other dimension of the multi-dimensional tensor network to cascade the different layers accordingly.

In some embodiments, the method further comprises decomposing the second many-body problem into a multi-dimensional tensor network.

In some embodiments, the first and/or second fermionic system(s) are/is one of: a predetermined molecule, a predetermined chemical reaction, and a predetermined chain reaction.

Some non-limiting examples of molecules could be mercury (Hg) complexes and organic molecules such as $C_3H_6O$, for which it is extremely difficult to calculate stability properties from first principles. Some non-limiting examples of chemical reactions could be the bond dissociation of molecules (such as $H_2$ -> H + H and alike) and exchange reactions (such as $H_2$ + H -> H + $H_2$). Some non-limiting examples of chain reaction could be any concatenation of the previous reactions.

In some embodiments, the multi-dimensional tensor network is representable with a projected entangled-pair state (PEPS), a tree tensor network (TTN) and/or a multi-scale entanglement renormalization ansatz (MERA).

PEPS, TTN and/or MERA in some cases simplify the processing of the tensor networks to apply the considerations of fermionic systems, particularly at least the consideration of the anti-commutation since the crossings of legs of tensors are more easily identifiable and modifiable to introduce the sign swap tensors.

In some embodiments, the machine learning routine comprises a neural network based on a tensor network.

The neural network can be, for instance but without limitation, a tensor neural network such as the one disclosed in US patent application no. 17/729,575, which is incorporated by reference in its entirety herein. Such neural networks are related to tensor networks by having layers of tensors.

In some embodiments, the method further comprises training the machine learning routine with one or more sets of historical data associated with the first fermionic system of the predetermined machine learning routine.

The training can be of different kinds to improve the results it provides over time by adjusting the parameters thereof based on the training data. In some embodiments, the training is conducted via back-propagation and gradient descent algorithms, targeting the minimization of a loss function that can be of different types. In some other embodiments, the training is conducted variationally as in tensor network algorithms, sweeping throughout the tensors in the system, also minimizing a loss function.

In some embodiments, one, some or each tensor network defining a layer of the predetermined machine learning routine is a matrix product operator.

A set of matrix product operators, MPOs, is found in the tensor network defining the layers of the routine, and one or more layers are defined such that they are respective MPOs.

In some embodiments, the multi-dimensional tensor network is a two-dimensional tensor network, and some or each tensor network defining a layer of the predetermined machine learning routine is a matrix product operator.

In some embodiments, each many-body problem takes the form of a Hamiltonian or a Hubbard-like discrete Hamiltonian.

The Hamiltonian can take, for example but without limitation, the following form as known in the art:

$$\hat{H} = \frac{1}{2}\int_r \hat{\psi}_r^\dagger[-\nabla^2 + v(r)]\hat{\psi}_r + \frac{1}{2}\int_{rr'} u(r,r')\,\hat{\psi}_r^\dagger\hat{\psi}_{r'}^\dagger\hat{\psi}_{r'}\hat{\psi}_r$$

The Hubbard-like discrete Hamiltonian can take, for example but without limitation, the following form as known in the art:

$$H = \sum_{ij} t_{ij} \hat{c}_{i\sigma}^\dagger \hat{c}_{j\sigma} + \sum_{ijkl} V_{ijkl} \hat{c}_{i\sigma}^\dagger \hat{c}_{j\sigma'}^\dagger \hat{c}_{k\sigma'} \hat{c}_{l\sigma}$$

with:

$$t_{ij} = \int_r \phi_i(r) \left[ -\frac{1}{2}\nabla^2 + v(r) \right] \phi_j(r)$$

$$V_{ijkl} = \int_{r_1, r_2} \frac{\phi_i(r_1)\phi_j(r_2)\phi_k(r_2)\phi_l(r_1)}{|r_1 - r_2|}$$

In some embodiments, the method further comprises processing one or more Hamiltonians associated with each fermionic systems to provide a respective many-body problem in the form of a Hubbard-like discrete Hamiltonian.

A second aspect of the disclosure relates to a data processing apparatus or system comprising means for carrying out the steps of a method as disclosed in the first aspect.

The data processing apparatus or system, the latter including a plurality of data processing apparatuses, execute the computer-implemented method for establishing a machine learning routine capable of providing data/information about a fermionic system that is modelled by a many-body problem.

Each data processing apparatus is a computing apparatus comprising at least one processor and at least one memory, the at least one memory being configured, together with the at least one processor, to make the data processing apparatus (or the data processing system) to e.g.: process a predetermined machine learning routine in the form of a tensor network defining layers of tensors in the routine, the predetermined machine learning routine being adapted for a regression problem of fermionic systems that are molecules or chemical reactions, the predetermined machine learning routine being processed such that: each tensor of the tensor network of the predetermined machine learning routine is converted into a parity preserving tensor; and a sign swap tensor is introduced in the tensor network at each crossing of legs of different tensors in the tensor network of the predetermined machine learning routine; inputs a first many-body problem modeling a first fermionic system in the processed predetermined machine learning routine; and outputs from the processed predetermined machine learning routine at least one result for the first fermionic system after having inputted the first many-body problem, with the at least one result being inferred by the processed predetermined machine learning routine.

A third aspect of the disclosure relates to a computer program comprising instructions which, when the program is executed by at least one computing device, cause the at least one computing device to carry out the steps of a method as disclosed in the first aspect.

In some embodiments, the computer program is embodied on a non-transitory computer-readable storage medium storing the computer program.

A fourth aspect of the disclosure relates to a data carrier signal carrying a computer program as described in the third aspect.

## BRIEF DESCRIPTION OF THE DRAWINGS

To complete the description and in order to provide for a better understanding of the disclosure, a set of drawings is provided. Said drawings form an integral part of the description and illustrate embodiments of the disclosure, which should not be interpreted as restricting the scope of the disclosure, but just as examples of how the disclosure can be carried out. The drawings comprise the following figures:

Figure 1 shows a computing apparatus or system in accordance with embodiments.
Figure 2 shows a computer-implemented method in accordance with embodiments.
Figures 3A-3B show a tensor and its Hermitian conjugate.
Figures 4A-4B show the incorporation of fermionic swaps.
Figures 5A-5B show parity preserving tensors.

Figures 6A, 6B, 7 and 8 show exemplary machine learning routines in the form of tensor neural networks as used in some embodiments.

## DETAILED DESCRIPTION

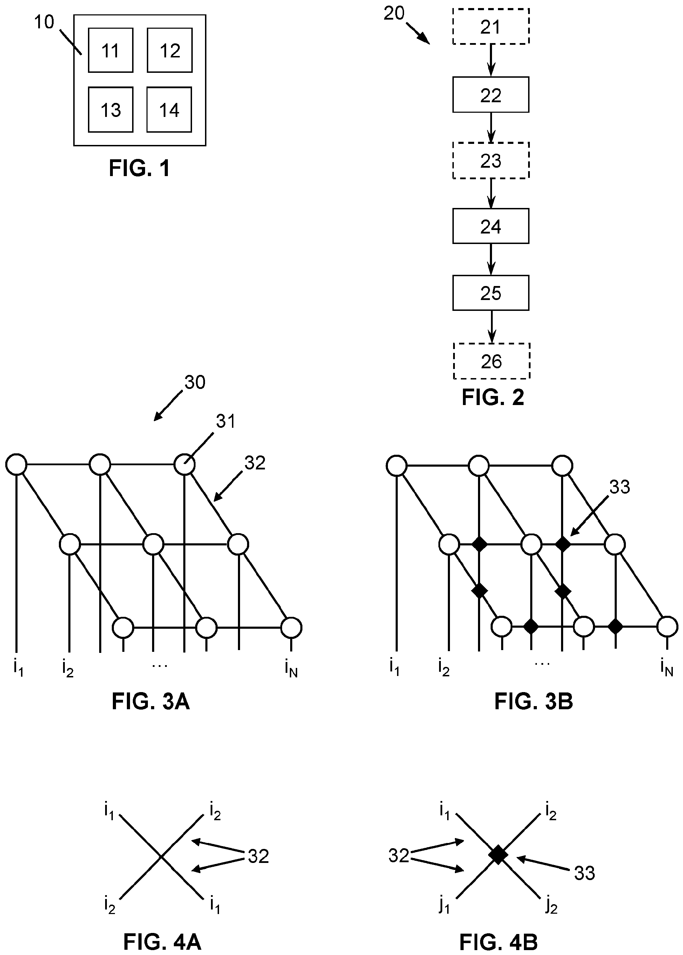

Figure 1 shows a computing apparatus or system 10 in accordance with embodiments. Methods according to the present disclosure can be carried out by such an apparatus or system 10, and computer programs can be run and computer-readable medium storages according to the present disclosure can be read by such an apparatus or system 10.

The apparatus or system 10 comprises at least one processor 11 and at least one memory 12.

The apparatus or system 10 also comprises, in some embodiments, a communications module 13 at least configured to receive data from and transmit data to other apparatuses or systems in wired or wireless form, thereby making possible to e.g. receive many-body problems in the form of electrical signals, either in analog form, in which case the apparatus or system 10 digitizes them, or in digital form, and to e.g. transmit control commands. The apparatus or system 10 additionally or alternatively comprises, in some embodiments, at least one input port for receiving electrical signals of a user input device (such as a keyboard, a touchpad, a touchscreen, etc.), e.g. for introduction of many-body problems.

Figure 2 shows a computer-implemented method 20 in accordance with embodiments.

The method 20 comprises, in some embodiments (visually represented with the dashed-border block), a step whereby the computing apparatus or system provides a predetermined machine learning routine or algorithm by processing a many-body problem modeling a fermionic system, such as a molecule, a chemical reaction or a chain reaction. Such many-body problem is decomposed into a multi-dimensional tensor network, or is decomposable, in which case the method 20 also comprises a step of decomposing the many-body problem into the multi-dimensional tensor network.

The method 20 comprises a step 22 whereby a computing apparatus or system processes a predetermined machine learning routine or algorithm, for example a neural network, to convert it into a routine for more accurately processing problems related to a fermionic system. The routine is structured as a tensor network and, thus, has tensors, typically tensor networks, as layers. The routine is such that is adapted for regression problems of fermionic systems that are molecules, chemical reactions or chain reactions. The conversion is such that the tensors of each layer are converted into symmetric tensors, and fermion swaps in the form of sign swap tensors are provided in the machine learning routine at each crossing of legs of different tensors.

In those embodiments in which the method 20 comprises step 21, the predetermined machine learning routine or algorithm processed 22 is that provided in step 21. In other embodiments, the predetermined machine learning routine or algorithm is an existing one, e.g. one routine or algorithm stored, retrieved from a server, etc.

The method 20 comprises, in some embodiments, a step 23 whereby the computing apparatus or system trains the processed machine learning routine with one or more sets of historical data preferably associated with the fermionic system of the machine learning routine or algorithm.

The method 20 comprises a step 24 whereby the computing apparatus or system inputs in the processed machine learning routine or algorithm a many-body problem modeling a fermionic system, particularly a molecule, a chemical reaction or a chain reaction, preferably the same type of fermionic system that the routine is configured for.

The method 20 comprises a step 25 whereby the computing apparatus or system outputs at least one parameter inferred by the processed machine learning routine following the input 24 of the many-body problem.

The method 20 comprises, in some embodiments, a step 26 whereby the computing apparatus or system processes one or more parameters outputted 25 by the machine learning routine upon input 24 of the many-body problem to determine a possible future state of the concerned fermionic system, and/or derive a configuration for the concerned fermionic system to alter its dynamics or operation, and/or compare one or more parameters with one or more predetermined thresholds for selecting one or more predetermined commands based on the result of comparison and, optionally, transmitting the command(s) to at least one control device associated with the concerned fermionic system.

Figures 3A-3B show the incorporation of fermionic swaps with the aid of a PEPS representation.

A multi-dimensional tensor network 30 can be represented with PEPS as known in the art, as illustrated in Figure 3A. The tensor network includes a plurality of N tensors 31 and legs 32 associated therewith. The PEPS representation may simplify the identification of legs' crossings.

As part of the definition of the machine learning routine in embodiments of the present disclosure, as illustrated in Figure 3B a sign swap tensor 33 is arranged at each legs' crossing. The addition of such tensors 33 implements the anticommutation fermionic operators.

Figures 4A-4B show the incorporation of fermionic swaps in the crossing of legs of tensors not shown.

In Figure 4A, the legs 32 of two tensors, e.g. tensor $i_1$ and tensor $i_2$, cross each other. According to the aforementioned procedure, a fermionic swap gate/tensor 33 is provided at the crossing. The illustrated tensor 33 can be e.g. fermionic swap tensor $X_{i_2 i_1 j_1 j_2}$:

$$X_{i_2 i_1 j_1 j_2} = \delta_{i_1,j_2} \delta_{i_2,j_1} S(i_1, i_2),$$

with $S(i_1, i_2)$ given by

$$S(i_1, i_2) \equiv \begin{cases} -1 & \text{if } p(i_1) = p(i_2) = -1 \\ 1 & \text{otherwise} \end{cases}$$

with p being the eigenvalue of the parity operator P for each tensor index.

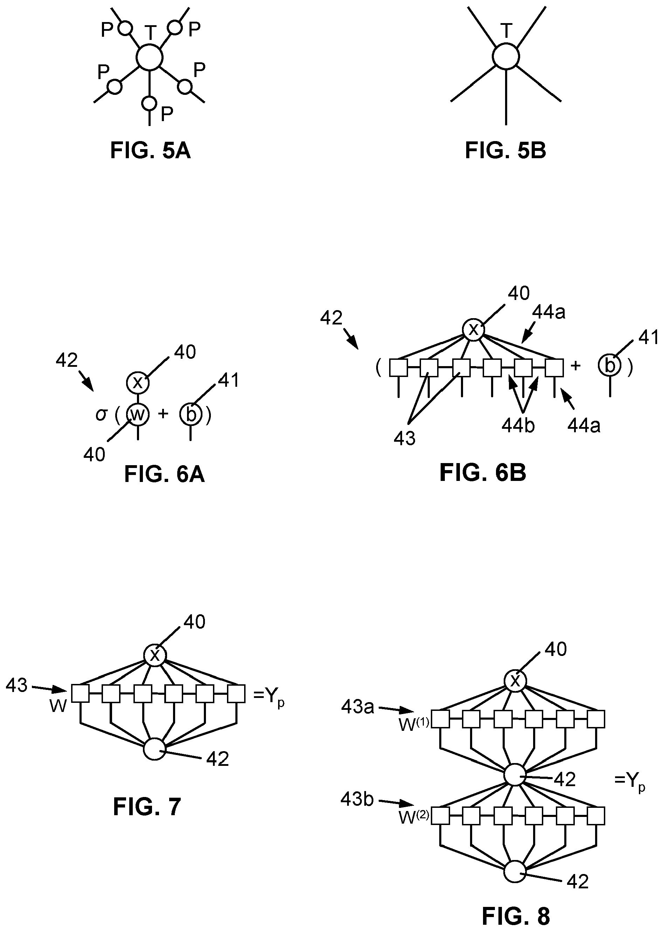

Figures 5A-5B show parity preserving tensors T.

The parity preserving tensors T have parity operators P, as illustrated in Figure 5A, acting on the indices of the tensor, thereby rendering the tensor T illustrated in Figure 5B.

Figures 6A and 6B show, in different visual formats, an exemplary tensor neural network as a possible machine learning routine or algorithm used in some embodiments.

Tensor neural networks have similarities with respect to classical neural networks. The tensor neural networks also have an input vector 40 that can be provided in the form of a tensor. The tensor neural networks also have an activation function 42 (visually represented in Figures 7 and 8) that depends upon a tensor network in the form of an MPO 43 and a bias 41, if any, that is added to the MPO 43 and which may also be present in classical neural networks.

Each tensor network or MPO 43 of the tensor neural network is equivalent to a hidden layer of a classical neural network. Each tensor of an MPO 43 includes two external indices or physical dimensions 44a linked to the input and output of the tensor network, and two internal indices or virtual dimensions 44b linked to neighboring tensors, the only exception being the tensors at the edges that only have one internal index or virtual dimension 44b.

Figures 7 and 8 show different tensor neural network as a possible machine learning routine or algorithm used in some embodiments. Particularly, Figure 7 shows a single layer tensor neural network due to its only hidden layer in the form of an MPO 43, and Figure 8 shows a multiple layer tensor neural network to its two hidden layers in the form of respective MPOs 43a, 43b.

Each MPO 40 has an activation function 42 associated therewith. In these examples, the activation function 42 is a linear rectifier, but it will be noted that any activation function 32 known in the art could be used instead without departing from the scope of the present disclosure.

As illustrated in each of Figures 7 and 8, the predicted value of each tensor neural network for the given tensor networks is said to be $Y_p$. The predicted value $Y_p$ can be computed as the contraction of the complete tensor networks, which is convenient for simplified training of the neural networks.

In this text, the term "includes", "comprises" and derivations thereof (such as "including", "comprising", etc.) should not be understood in an excluding sense, that is, these terms should not be interpreted as excluding the possibility that what is described and defined may include further elements, steps, etc.

On the other hand, the disclosure is obviously not limited to the specific embodiment(s) described herein, but also encompasses any variations that may be considered by any person skilled in the art (for example, as regards the choice of components, configuration, etc.), within the general scope of the invention as defined in the claims.

**Claims**

1. A computer-implemented method comprising:

    processing a predetermined machine learning routine in the form of a tensor network that defines layers of tensors in the routine, the routine being adapted for a regression problem of fermionic systems that are molecules or chemical reactions, the routine being processed such that:

        each tensor of the tensor network of the predetermined machine learning routine is converted into a parity preserving tensor; and
        a sign swap tensor is introduced in the tensor network at each crossing of legs of different tensors in the tensor network of the predetermined machine learning routine, thereby implementing anticommutation fermionic operator;

    inputting a first many-body problem modeling a first fermionic system in the processed predetermined machine learning routine, the first fermionic system being a molecule or a chemical reaction; and
    outputting from the processed predetermined machine learning routine at least one parameter for the first fermionic system after having inputted the first many-body problem, the at least one parameter being inferred by the processed predetermined machine learning routine.

2. The computer-implemented method of claim 1, wherein the at least one parameter comprises one or more of: a potential of a molecule of the first fermionic system, a velocity of a chemical reaction of the first fermionic system, dynamics of the first fermionic system, and one or more characteristics of at least one element taking part in the first fermionic system.

3. The computer-implemented method of claim 2, further comprising processing the at least one parameter to:

   estimate a future state of the first fermionic system; and/or
   derive a configuration for the first fermionic system that alters dynamics thereof in a predetermined manner; and/or
   compare the at least one parameter with one or more predetermined thresholds, and output at least one predetermined command based on the comparison.

4. The computer-implemented method of claim 3, wherein the at least one parameter is processed so that at least the predetermined command is outputted, wherein the at least one predetermined command is transmitted to a control device associated with the first fermionic system.

5. The computer-implemented method of claim 4, further comprising, after transmitting the at least one predetermined command, inputting in the processed predetermined machine learning routine a second many-body problem modeling the first fermionic system upon at least one change is introduced or is to be introduced by the control device so that the processed predetermined machine learning routine outputs at least another parameter comprising one or more of: a potential of a molecule of the first fermionic system with the change, a velocity of a chemical reaction of the first fermionic system with the change, dynamics of the first fermionic system with the change, and one or more characteristics of at least one element taking part in the first fermionic system with the change.

6. The computer-implemented method of any one of the preceding claims, further comprising providing the predetermined machine learning routine by processing a second many-body problem modeling a second fermionic system, wherein the second many-body problem is decomposed or is decomposable into a multi-dimensional tensor network.

7. The computer-implemented method of claim 6, further comprising decomposing the second many-body problem into a multi-dimensional tensor network.

8. The computer-implemented method of any one of the preceding claims, wherein each fermionic system is one of: a predetermined molecule, a predetermined chemical reaction, and a predetermined chain reaction.

9. The computer-implemented method of any one of the preceding claims, wherein the machine learning routine comprises a neural network based on a tensor network.

10. The computer-implemented method of any one of the preceding claims, further comprising training the machine learning routine with one or more sets of historical data associated with the fermionic system of the predetermined machine learning routine.

11. The computer-implemented method of any one of the preceding claims, wherein one, some or each tensor network defining a layer of the predetermined machine learning routine is a matrix product operator.

12. The computer-implemented method of claim 11, wherein the multi-dimensional tensor network is a two-dimensional tensor network, and wherein each tensor network defining a layer is a matrix product operator.

13. The computer-implemented method of any one of the preceding claims, wherein each many-body problem takes the form of a Hamiltonian or a Hubbard-like discrete Hamiltonian.

14. A data processing apparatus or system comprising means for carrying out the steps of the method of any one of the preceding claims.

15. A computer program comprising instructions which, when the program is executed by at least one computing device, cause the at least one computing device to carry out the steps of the method of any one of claims 1-13.

FIG. 1

FIG. 2

FIG. 3A

FIG. 3B

FIG. 4A

FIG. 4B

**FIG. 5A**

**FIG. 5B**

**FIG. 6A**

**FIG. 6B**

**FIG. 7**

**FIG. 8**

Europäisches
Patentamt

European
Patent Office

Office européen
des brevets

## EUROPEAN SEARCH REPORT

Application Number

EP 22 38 3137

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Schneider M. ET AL: "The Hubbard model on a honeycomb lattice with fermionic tensor networks", arxiv.org, Cornell University, 23 December 2021 (2021-12-23), pages 1-9, XP093043027, DOI: 10.48550/arXiv.2110.15340 Retrieved from the Internet: URL:chrome-extension://efaidnbmnnnibpcajpc glclefindmkaj/https://arxiv.org/pdf/2110.1 5340.pdf [retrieved on 2023-04-28] * the whole document * | 1-15 | INV. G16C20/30 G16C20/70 |
| X | CARRASQUILLA J.: "Machine learning for quantum matter", ADVANCES IN PHYSICS: X, vol. 5, no. 1, 1 January 2020 (2020-01-01) , page 1797528, XP093043028, DOI: 10.1080/23746149.2020.1797528 * the whole document * | 1-15 | |
| | | | TECHNICAL FIELDS SEARCHED (IPC) |
| X | ORUS R.: "Tensor networks for complex quantum systems", NATURE REVIEWS PHYSICS, vol. 1, 5 August 2019 (2019-08-05), pages 538-550, XP093043030, DOI: 10.1038/s42254-019-0086-7 * the whole document * | 1-15 | G16C G06N |

The present search report has been drawn up for all claims

| Place of search | Date of completion of the search | Examiner |
|---|---|---|
| The Hague | 29 April 2023 | Godzina, Przemyslaw |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 729575